# EUROPEAN PATENT APPLICATION

(11) **EP 1 748 373 A2**
(43) Date of publication of application: **31.01.2007**
(21) Application number: 06115244.3
(22) Date of filing: 09.06.2006
(51) Int. Cl.: G06F 19/00

(54) **A method for processing and displaying sequences of graphical symbols in a colour code, and relevant representation on supports**

(30) Priority: 13.07.2005 IT BS20050087
(71) Applicant: Universita' degli studi di Brescia, 25121 Brescia (IT)
(72) Inventor: Barlati, Sergio, 25133 BRESCIA (IT); Barlati, Stefano, 25133 BRESCIA (IT); Borsani, Giuseppe, 20090 SEGRATE (Milano) (IT); Barbon, Alessandro, 36012 ASIAGO (Vicenza) (IT)
(74) Representative: Crippa, Paolo Ernesto

(57) **Abstract**

A method for processing and displaying sequences of graphical symbols in a colour code, which allows obtaining a compact and immediate representation of the meaning of the symbols themselves. Such representations are preferably obtained with different combinations or chromatic maps so as to be compact and quickly understandable. Moreover, such representations may be represented on supports of various types, among which for example printed, woven or spun surfaces.

## Description

The present invention relates to a method for processing and representing sequences of graphical symbols of various types, such as letters of the alphabet or music notes, so as to group, process and display the information correlated to the graphical symbols themselves. The present invention also relates to the graphical reproduction on supports of various types of such sequences of symbols in different formats, so as to obtain a clear and compact display of such information. In particular, the invention relates to a method for manufacturing fabrics that represent sequences of graphical symbols suitably processed in chromatic codes.

The present invention finds an advantageous application in the analysis and representation of graphical symbols resulting from sequences of DNA-related letters.

In the art, the need of analysing complex sequences of symbols for obtaining a compact representation therefrom, so as to simplify the study of the meanings correlated to the sequences and to the symbols themselves.

The object of the present invention is to provide a method for processing and representing sequences of graphical symbols of various types capable of solving the problems mentioned above with reference to the prior art. The object of the present invention is also to graphically represent such information on supports of various types, among which for example fabrics.

Such object is achieved by a processing and representation method according to claim 1.

Further features and the advantages of the processing and representation method according to the present invention will appear from the following description of a preferred non-limiting embodiment thereof, wherein:

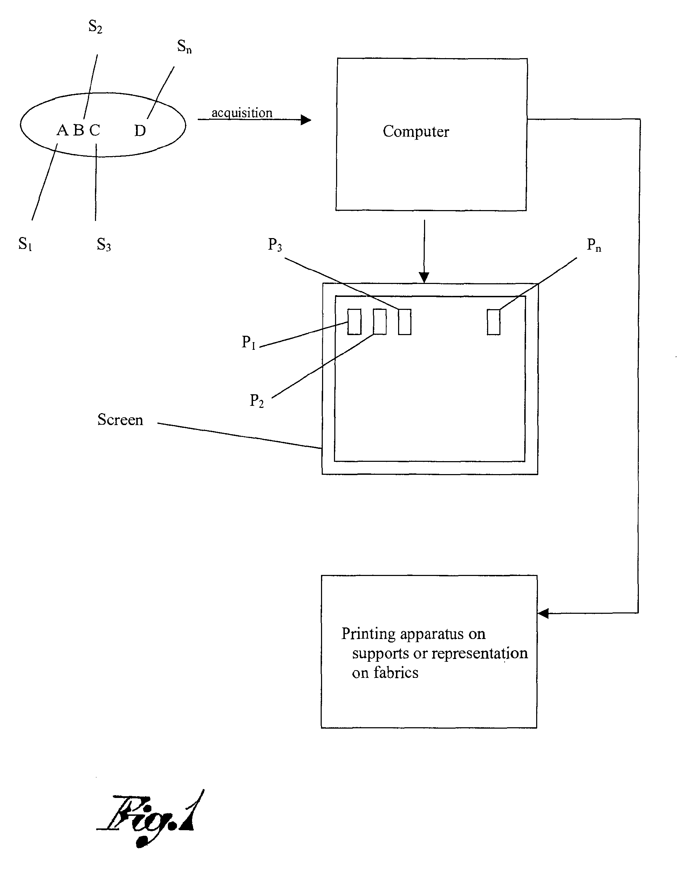

figure 1 shows a system suitable for carrying out the method according to the present invention;

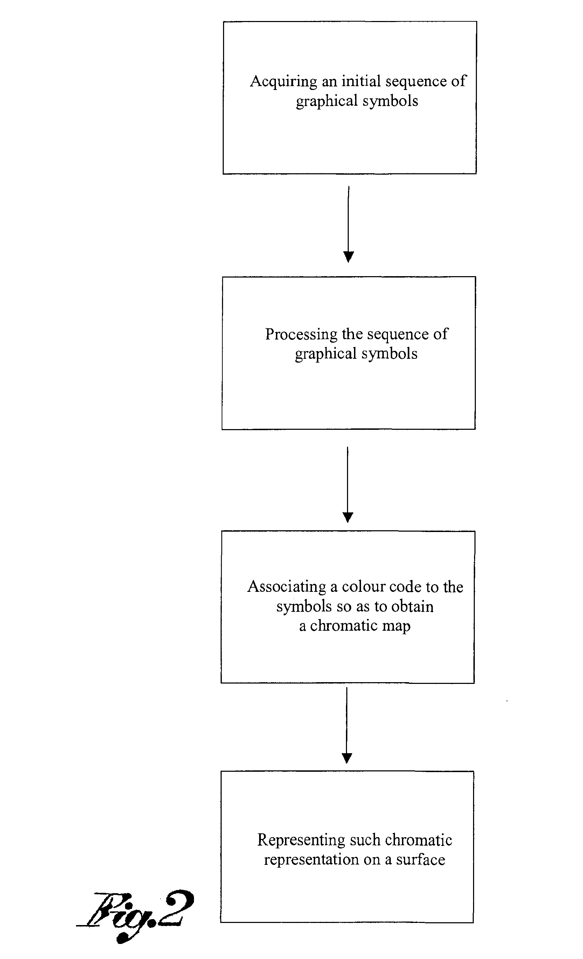

figure 2 shows a flow chart of the method according to the present invention;

figure 3 shows a view of a possible graphical representation of a sequence of symbols, obtained with the method according to the invention;

figure 4 shows a view of a further possible graphical representation of a sequence of symbols, obtained with the method according to the invention;

figure 5 shows a view of a further possible graphical representation of a sequence of symbols, obtained with the method according to the invention.

In the first place, the present invention relates to a method for analysing and displaying sequences of symbols Sᵢ of any type, such as letters, music notes and the like.

In particular, it relates to a method for transforming any document or information coded into letters or in general, into symbols Sᵢ, into sequences of colours selected as desired.

According to an embodiment, the sequences of symbols Sᵢ to be displayed as colour codes may be DNA sequences derived from any genome, wherein the nitrogenous bases of the DNA are conventionally represented with letters 'A,T,C,G'.

Moreover, it is possible to represent as colour codes the sequences of proteins coded from the respective 20 amino acids forming them, wherein each amino acid corresponds to a specific letter of the alphabet.

Moreover, it is possible to represent sequences Sᵢ of any type of text in any language, wherein for example the letters or symbols of the alphabet can be mapped with different colours, including punctuations, spaces and any other graphical symbol present in the text. In general, it is possible to represent as colour codes any sequence of symbols Sᵢ having a meaning, such as notes and symbols of the scores of music works of any kind.

IN the specific case of DNA sequences, it is possible to transform the complete genome of any organism in colour code.

The processing method is preferably carried out with the aid of a computer and relevant software.

The computer program may comprise a plurality of functions intended for representing the contents of symbols Sᵢ but also for their re/processing, according to specific needs.

According to an embodiment, in a first step of acquisition of data Sᵢ, such program is capable of acquiring and processing sequences of symbols Sᵢ of any type, not just sequences related to nucleotides.

The step of acquiring the sequences of symbols Sᵢ can take place according to different methods.

For example, it is possible to establish a connection to the Internet, for example to the program "UCSC Genome Browser", to enable the online acquisition of the genomic sequences. It is in any case possible through the Internet to acquire any sequence of symbols, both of the text type and of the graphical type.

The program is capable of dividing the sequences of symbols Sᵢ to be loaded into block of desired dimensions, so as to group them into blocks of standard dimensions.

The data acquisition can take place through external storage devices, such as floppy disks, or other portable memories, or it is possible to directly input the data to the program itself or copy and move the data from other memories of the program or computer.

The sequence of symbols Sᵢ may comprise symbols of any type, among which letters of the alphabet, optionally spaced from one another; also the space between two symbols of consecutive groups of symbols can in turn be regarded as a symbol.

The program is therefore capable, once the data have been acquired according to one of the methods mentioned above, of processing the same to then represent them in a compact manner.

In the processing step, the program also enables search and comparison of symbols within the sequence, so as to locate and recognise the presence of a symbol or group of symbols within the sequence loaded. It is possible to compare one sequence with another one present for example on an internal or external memory or on an Internet website.

Moreover, it is possible to add one or more symbols in a selected sequence, inserted directly or taken from an external file.

The program provides a plurality of information on the sequence being analysed, such as the total number of elements that make up the sequence, the number of elements displayed on each line of the coloured sequence, the recurrence of an element in a sequence or portion of sequence.

The method therefore comprises the step of associating each symbol to a predetermined colour, which in any case can be changed in any step of the method. The mapping between a symbol and a colour may even not be biunique; in other words, a type of symbol, repeated any number of times in the sequence, is mapped to a given colour, but a same colour, for other purposes, may also be associated to two or more different symbols.

In the specific case of applications on DNA, it is possible to set the colour that will be assigned by default to each element of the genomic sequences or the colour that will be assigned to each element of the proteic sequences. Similarly, it is possible to set the colour that will be assigned by default to the characters in the text sequences.

The method comprises the step of associating each symbol in the sequence with a corresponding graphical element Pᵢ of a screen connected to the processor and of activating or lighting such graphical element Pᵢ with the colour selected for that same symbol.

According to an embodiment, said graphical element Pᵢ matches a pixel of said screen of the computer.

Preferably, the method provides for the activation and lighting of pixels Pᵢ with the selected colours, following a sequential filling pattern by lines of the screen, filling such lines from left rightwards, starting from the first line on top of the screen.

Once a line has been completed, at the right end, the method provides for continuing with filling the subsequent line below, starting from the left end thereof. Of course, it is possible to provide for other filling patterns of pixels Pᵢ, for example by columns.

The chromatic map obtained can be converted at any time into the original symbol map; that is, the software allows switching from the display of the colour sequence to the display of the symbol sequence when desired.

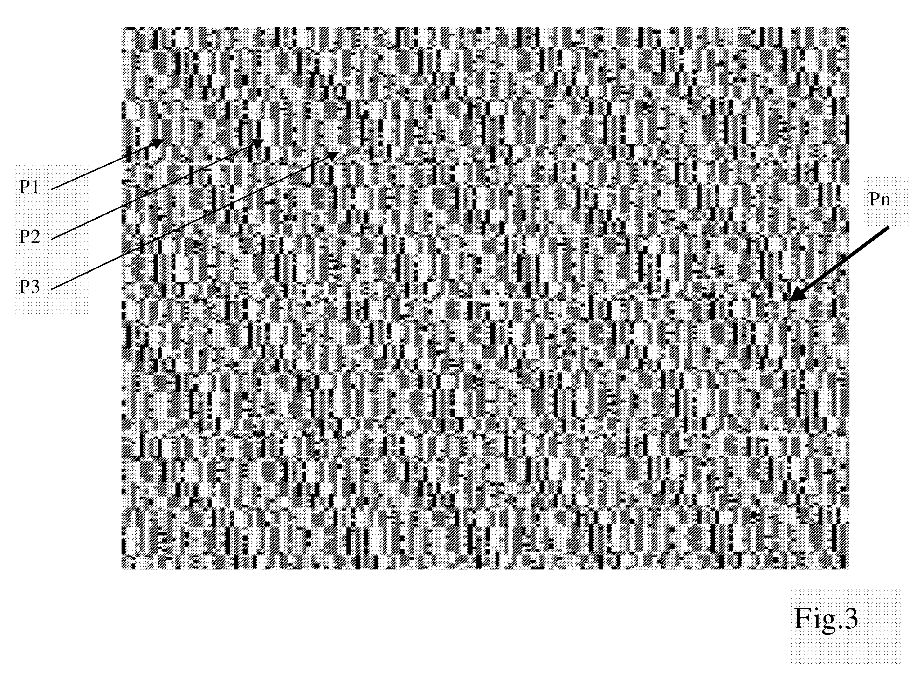

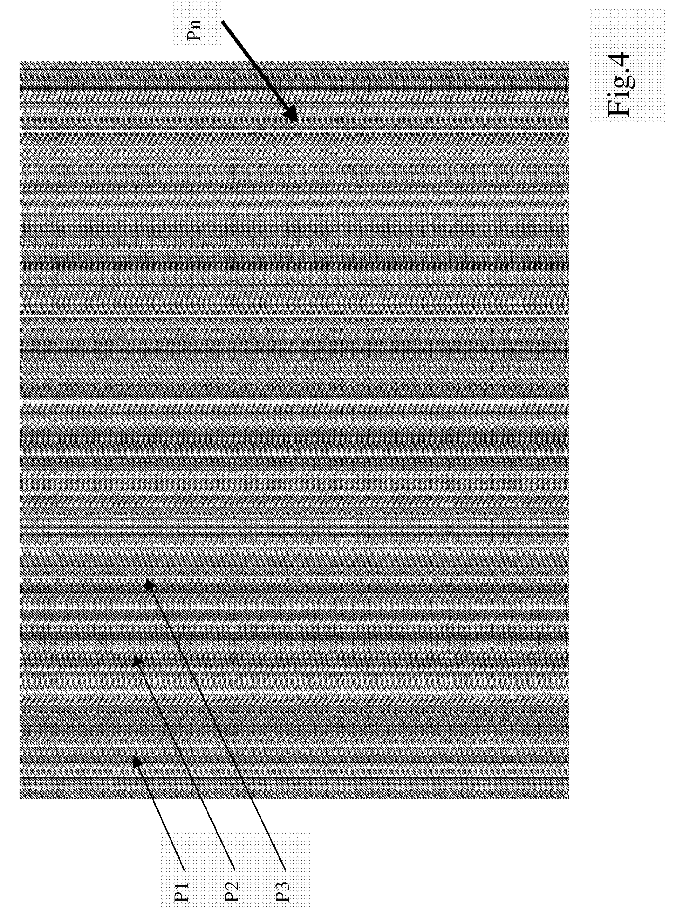

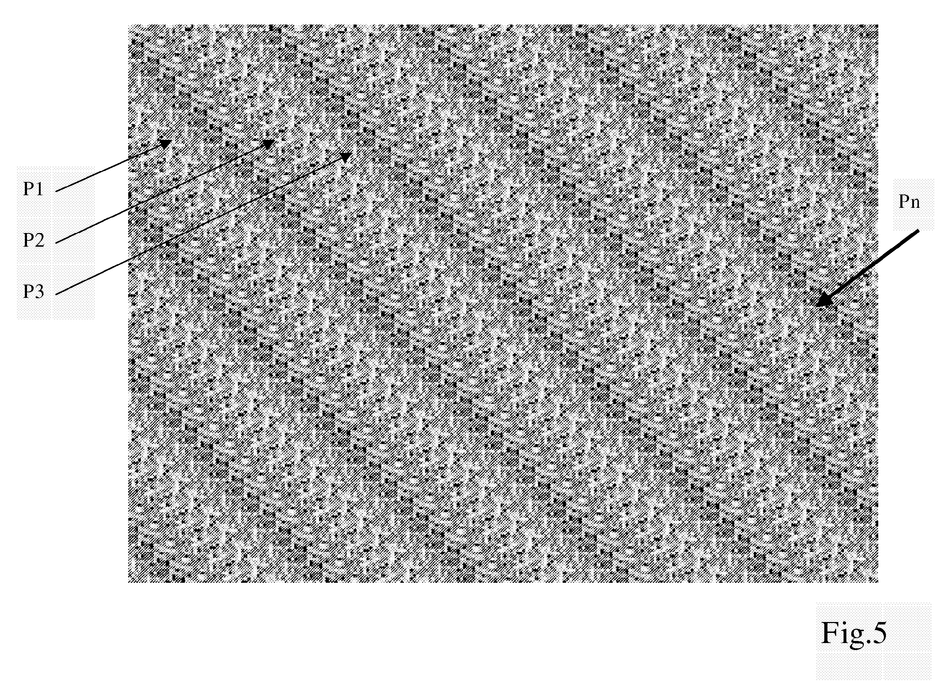

The display may be limited to a symbol or a group of selected symbols. Three different examples of display of chromatic maps starting from a sequence of symbols can for example be seen in figures 3-5, which show three different screens obtained by processing sequences of symbols and displaying the same with chromatic maps, by the method according to the present invention.

It is further possible to display the chromatic or symbol differences between two selected sequences of symbols.

The software allows enlarging or reducing the display window of the colour sequence to better highlight the dimension and the features of the analysed sequences. It is further possible to remove one or more sequences corresponding to the genes and/or non-coding sequences.

Moreover, it is possible to change the dimension of the pixel by element, that is, change the dimension with which the elements are outlined.

The present invention further relates to a method for obtaining a graphical representation of a sequence of symbols on a support. In other words, once a sequence of symbols has been acquired and after having sorted and optionally processed the same in a colour code according to the method described above, it is possible to transfer the chromatic map thus obtained on a support.

The support may be of any type, for example paper, and preferably it is a fabric. The support shows at least partly a chromatic map obtained by applying the method according to the invention, so as to represent a map having a meaning directly relatable to the sequence of symbols from which it has been generated.

The method preferably comprises the use of the computer for making the graphical representation of the chromatic maps obtained, preferably using fabrics as support.

For example, the computer may be connected to an apparatus or machinery for manufacturing fabrics, so as to produce a fabric that represents at least partly a chromatic scheme processed starting from an initial sequence of symbols.

Advantageously, the fabric thus obtained represents both the sequence and the colour and layout of the pixels as they are displayed on the screen.

Of course, it is possible to represent the chromatic map thus obtained on any surface of any material.

The graphical representation on a surface may also not exactly reflect the representation obtained on the screen following the processing of the initial sequence of symbols. In other words, it is preferable that the selection and the chromatic sequence are reflected, but not necessarily the shape of the single graphical elements or pixels.

Therefore, if the geometry of the single pixels is square or rectangular, it is possible to represent the same chromatic map for example on a fabric, but using circular graphical elements or of any other shape. Reflecting the sequence and the chromatic layout of the graphical elements maintains the meaning of the representation of the graphical symbols, but changing the shape of the single graphical elements can impart a different visual effect to the entire chromatic map.

As can be understood from the description, the methods according to the invention allow overcoming the disadvantages of the prior art.

In fact, the method allows acquiring, sorting and representing in a compact manner a plurality of information acquired in the form of symbols.

In the specific case of DNA, the advantage is to display in a single screen (for example, a page of a PC screen) a sequence of about 1 million bases. So, for example, the human genome, which comprises over 3 billion bases, can be displayed completely, compressed, in about 3000 pages or screens.

The software allows having an overall view of long portions of the genome and identifying recurring reasons for trying to associate a specific function thereto.

The present invention can have a plurality of applications.

Besides the scientific fields of particular interest for the study of the genome and for the comparative study of the genomes of different organisms, the software can in fact find application in all the fields wherein it is possible to use a chromatic information for decorative and information transmission purposes.

Below are some examples, such as the production of fabrics whose colour pattern has the information required (sequence of a gene, insertion of a phrase...) .

In the specific case of gene, the fabric is matched with the information relating to the gene, its functions and properties. This is also intended for promoting biological and genetic knowledge that are increasingly becoming a cognitive need in current life.

It is further possible to produce printed matter for fabrics used in clothing and at home, such as T-shirts, towels, foulards, ties, scarves, or any other type of article of clothing, but also blankets, curtains and so on.

It is possible to produce covers for notebooks, diaries and/or books of any type, especially useful for promoting biologic knowledge in schools.

It is possible to make coatings for motor vehicles or public means that represent, at least partly, chromatic maps obtained by acquiring and processing sequences of graphical symbols of any type.

It is possible to produce items of any type, such as cups, plates, lampshades, crockery, containers, but also glass walls, advertising posters or any other equivalent product used in building, furnishing, graphics.

It is possible to produce logos, initials of names of industries, companies that want to use the principle of the colour code to apply for example to the name of the company they represent.

It is proposed to use the colour code for the production for example of gadgets, ties, scarves and the like that have for example the name of an industry or company has meaning.

Of course, a man skilled in the art may make several changes and adjustments to the method for processing graphical symbols described above in order to meet specific and incidental needs, all falling within the scope of protection defined in the following claims.

## Claims

1. A method for processing graphical symbols, comprising the steps of:
- acquiring an initial sequence of graphical symbols;
- processing said sequence of graphical symbols;
- associating a colour code to said symbols so as to obtain a chromatic map based on said initial sequence of graphical symbols;
- transferring at least partly said chromatic representation on a surface.

2. A method according to claim 1, wherein the step of acquiring the graphical symbols comprises the use of a computer program suitable for acquiring and storing sequences of graphical symbols of any type.

3. A method according to claim 1 or 2, wherein said program acquires sequences of graphical symbols through online information network.

4. A method according to claim 2 or 3, wherein said program divides the sequences of symbols acquired into block of desired dimensions, so as to group the symbols into blocks of predetermined dimensions.

5. A method according to any one of claims 2 to 4, wherein the data acquisition takes place through external storage devices, such as floppy disks, or other portable memories, or through direct input of the data to the program itself or the copy and move of the data from other memories of the program or computer.

6. A method according to any one of the previous claims, wherein the step of processing the sequence of graphical symbols comprises the step of search and comparison of symbols within the sequence, so as to locate and recognise the presence of a symbol or group of symbols within the sequence loaded.

7. A method according to any one of the previous claims, wherein the processing step comprises the step of adding one or more symbols in a selected sequence, inserted directly or taken from an external file.

8. A method according to any one of the previous claims, wherein in said processing step the program provides a plurality of information on the sequence being analysed, such as the total number of elements that make up the sequence and the recurrence of an element in a sequence or portion of sequence.

9. A method according to any one of the previous claims, wherein the step of associating a colour code to said symbols comprises the step of selecting and associating a predetermined colour to each graphical symbol of said sequence.

10. A method according to claim 9, wherein the mapping between a symbol and a colour is not biunique so that a given symbol, repeated any number of times in the sequence, is mapped to a given colour, but a same colour may also be associated to two or more different symbols.

11. A method according to claims 9 or 10, wherein two or more groups of symbols having a predetermined meaning are associated to a given colour.

12. A method according to any one of the previous claims, wherein said graphical symbols are elements of genomic sequences or elements of proteic sequences.

13. A method according to any one of the previous claims, wherein the method comprises the step of associating each symbol in the sequence with a corresponding graphical element of a screen connected to the processor and of activating or lighting such graphical element with the colour selected for that same symbol.

14. A method according to claim 13, wherein each graphical element corresponds to a pixel of said screen.

15. A method according to any one of the previous claims, wherein the method provides for the activation and lighting of the graphical elements of the screen with the selected colours, following a sequential filling pattern by lines of the screen, filling such lines from left rightwards, starting from the first line on top of the screen.

16. A method according to claim 15, wherein during the pixel filling step, once a line has been completed, at the right end, the subsequent line below is filled, starting from the left end thereof.

17. A method according to any one of the previous claims, wherein the method provides for a step of filling the graphical elements by subsequent columns.

18. A method according to any one of the previous claims, wherein the method provides for the step of changing the dimension or the number of pixels by element, that is, changing the dimension or the shape of the graphical elements.

19. A method for manufacturing a product carrying a representation comprising the steps of:
- obtaining a chromatic representation from an initial sequence of graphical symbols by the method according to any one of claims 1 to 18,
- transferring at least partly said representation on a physical support.

20. A method for manufacturing a product according to claim 19, comprising the step of representing a representation obtained by the method according to any one of claims 1 to 18 on a fabric, so as to produce an object to associate the biological, literary, musical and similar meaning of the initial sequence of graphical symbols in colour code.
